# EUROPÄISCHE PATENTSCHRIFT

(11) **EP 1 721 965 B1**
(45) Veröffentlichungstag und Bekanntmachung des Hinweises auf die Patenterteilung: **11.01.2012**
(21) Anmeldenummer: 06450073.9
(22) Anmeldetag: 12.05.2006
(51) Int. Cl.: C12N 1/10, A61K 39/002, A23L 1/30, C12Q 1/68

(54) **Verfahren zur Herstellung und Anwendung von Protozoenkulturen**
Method for the preparation and use of protozoan cultures
Procédé pour la préparation et l'utilisation des cultures de protozoaires

(30) Priorität: 12.05.2005 AT 8152005
(43) Veröffentlichungstag der Anmeldung: 15.11.2006
(73) Patentinhaber: Veterinärmedizinische Universität Wien, 1210 Vienna (AT)
(72) Erfinder: Hess, Michael, 1020 Wien (AT)
(74) Vertreter: Sonn & Partner Patentanwälte

(56) Entgegenhaltungen:
- US-A- 5 824 537
- TAN KEVIN S W ET AL: "Blastocystis hominis: A simplified, high-efficiency method for clonal growth on solid agar" EXPERIMENTAL PARASITOLOGY, Bd. 96, Nr. 1, September 2000 (2000-09), Seiten 9-15, XP002390406 ISSN: 0014-4894
- CLARK C G ET AL: "Methods for cultivation of luminal parasitic protists of clinical importance" CLINICAL MICROBIOLOGY REVIEWS, WASHINGTON, DC, US, Bd. 15, Nr. 3, Juli 2002 (2002-07), Seiten 329-341, XP002992846 ISSN: 0893-8512
- FARRI T A: "A simple technique for preparing clone cultures of Entamoeba histolytica." TRANSACTIONS OF THE ROYAL SOCIETY OF TROPICAL MEDICINE AND HYGIENE. 1978, Bd. 72, Nr. 2, 1978, Seiten 205-206, XP002390407 ISSN: 0035-9203
- MCDOUGALD L R: "Blackhead disease (histomoniasis) in poultry: A critical review" AVIAN DISEASES, Bd. 49, Nr. 4, Dezember 2005 (2005-12), Seiten 462-476, XP009069336 ISSN: 0005-2086

## Beschreibung

Die Erfindung betrifft die Herstellung und Anwendung von Protozoenkulturen von *Histomonas meleagridis, Tetratrichomonas gallinarum* und *Blastocystis* sp..

Die Schwarzkopfkrankheit, Typhlohepatitis oder Histomoniasis bei Puten wurde von Smith (1895) zum ersten Mal beschrieben. Wesentliche Erkenntnisse zur Ätiologie konnten durch die Untersuchungen von Tyzzer (1920) gewonnen werden. In dieser Arbeit werden zum ersten Mal Flagellen und Pseudopodien, als morphologisches Hauptkriterium des Erregers, beschrieben, was den Erreger als Protozoen einstufte. Die Morphologie des Parasiten und das Vorkommen bei Puten führten zur Bezeichnung *Histomonas meleagridis.* Die Erkenntnis, dass der Parasit bei Hühnern ebenfalls vorkommen kann, führte zur Empfehlung diese Tierarten getrennt zu halten. Als weitere empfängliche Vogelarten werden insbesondere Perlhuhn, Fasan, Pfau und Wachtel beschrieben, während das Wassergeflügel als nicht empfänglich gilt (McDougald, 2003). Durch die Pathogenese und die hohe Prävalenz der Erkrankung bei Puten ist diese Spezies sicherlich als einer der empfänglichsten Wirte anzusehen.

In den letzten beiden Jahren kam es in Europa zu einem verstärkten Auftreten der Erkrankung. Hintergrund ist insbesondere das Verbot geeigneter Prophylaktika und Therapeutika, mit deren Hilfe die Erkrankung über Jahrzehnte gezielt bekämpft wurde. Nichtsdestotrotz wurde die prophylaktische Verwendung von Therapeutika bei Puten, insbesondere von Nifursol, Imidazolderivaten, wie Nitroimidazol und Dimetridazol, als Futterzusatzstoff in der Europäischen Union verboten (siehe EEC1798/1995, EEC2205/2001 und EEC1756/2002), da die Unbedenklichkeit dieser Stoffe (Karzinogenität und Genotoxizität) für den Verbraucher nicht auszuschließen ist. Auch das in Nordamerika zur Prophylaxe eingesetzte Nitarsone (4-Nitrophenylarsonic acid) besitzt in Europa keine Zulassung. In Folge dieses absoluten Prophylaxe- und Therapienotstandes mussten in verschiedenen Ländern Europas, nach Ausbruch der Erkrankung in Putenbeständen, in den letzten beiden Jahren Keulungsmaßnahmen durchgeführt werden (Hess et al., 2004).

Bei Legehennen verläuft die Erkrankung milder, durch die zunehmende Alternativhaltung kommt es aber auch hier zu einem vermehrten Auftreten (Hafez et al., 2001; Esquenet et al., 2003).

Grundlegende Arbeiten über die Vermehrung von *Histomonas meleagridis* in Zellkulturmedien wurden von Dwyer (1970) und später von Stepkowski (1978) durchgeführt. Als Grundlage dient beiden Autoren Kotmaterial oder Isolate aus Kot, die unter agnotobiotischen Bedingungen zu vermehren sind. Mithin ist eine Vermehrung des Erregers nur in einem entsprechenden Milieu möglich, was hauptsächlich durch die Begleitflora bestimmt wird.

Insgesamt haben obige Kulturen den Nachteil, dass sie genetisch nicht einheitlich sind, da sie aus mehreren Erregern ein und derselben Spezies bestehen. Darüber hinaus ist nicht gewährleistet, dass sich auch andere Protozoen, die morphologisch schwierig zu unterscheiden sind, in der Kultur befinden. Das Vorkommen von verschiedenen Protozoen im Darm des Geflügels ist vielfach beschrieben (McDougald, 2003), was diese Problematik nachdrücklich unterstreicht.

Als Folge der wenig charakterisierten Kulturen sind Ergebnisse von *in vitro* und *in vivo* Experimenten, in denen solche Kulturen verwendet werden, zurückhaltend zu beurteilen. Die verlässliche Testung von Substanzen, sowohl *in vitro* als auch *in vivo,* fordert eine genetisch einheitliche Kultur, um zu verhindern, dass bestimmte Effekte auf das Vorhandensein verschiedener Subpopulationen oder andere Protozoenspezies, zurückzuführen sind. Die Ergebnisse von Tierversuchen, die in jüngster Vergangenheit durchgeführt wurden, spiegeln diese Heterogenität wieder (Hu und McDougald, 2003; Hu et al., 2004; Landmann, 2004).

Die Heterogenität von Kulturen bedingt, dass bestimmte Subpopulationen phänotypisch dominieren, was falsche Rückschlüsse auf die generelle Empfindlichkeit eines Erregers gibt. Dieses Problem ist beispielhaft für den Erreger der Malaria, *Plasmodium falciparum,* beschrieben (Rosario, 1981; Trager et al., 1981; Burkot et al., 1984; LeBras et al., 1983). Als Konsequenz hiervon besteht die Gefahr, dass im Rahmen von *in vitro* Tests lediglich eine Selektion und Vermehrung von resistenten Stämmen erfolgt, was keinen Rückschluss auf die Empfindlichkeit eines Erregers zulässt. Hinweise über mögliche unterschiedliche Subpopulationen von *Histomonas meleagridis* gibt es aus der Arbeit von Gerbod et al. (2001). Die Autoren haben von 4 Isolaten Teile der ribosomalen RNA - dieser Genabschnitt wird oft für phylogenetische Analysen benutzt - sequenziert und eine leichte Heterogenität an einigen Positionen festgestellt. Die Autoren schließen daraus, dass dies ein Hinweis für unterschiedliche Subpopulationen sein könnte. Gesicherte Hinweise über Subpopulationen innerhalb einer der drei oben erwähnten Protozoenspezies gibt es von *Tetratrichomonas gallinarum,* wie von Cepicka et al. (2005) berichtet wird. Auch von *Blastocystis* sp. ist die genetische Vielfalt ausführlich dokumentiert, auch im Hinblick auf eine mögliche Zoonoseproblematik (Noel et al., 2003; Noel et al., 2003).

In Kulda et al. (1974) wurden Infektionsexperimente mit *Tetratrichomonas gallinarum* durchgeführt und dabei wurde mit einem Isolat die für *Histomonas meleagridis* typische Schwarzkopfkrankheit ausgelöst. Es wurde gezeigt, dass diese Infektionskultur von *Tetratrichomonas gallinarum* mit *Histomonas meleagridis* kontaminiert war, basierend auf der mikroskopischen Kotuntersuchung.

In Goedbloed and Bool (1962) wurden Puten sowohl mit einer Kultur von *Histomonas meleagridis* als auch *Tetratrichomonas gallinarum* infiziert und anschließend histologische Untersuchungen durchgeführt. Nach gemischter Infektion wurden beide Erreger mikroskopisch im Kot nachgewiesen. Dies zeigt die Gefahr auf, dass bei Re-Isolierung von Protozoenkulturen mehrere Kulturen isoliert werden, da ein selektives Medium nicht bekannt ist.

Bayon and Bishop (1937) beschreiben die Isolierung von *Histomonas meleagridis* aus der Leber eines Huhns, welches zur Diagnostik verwendet wurde. Aus dem Kot des Tieres wurde eine Mischung aus *Blastocystis, Trichomonas* und *Chilomastix* isoliert, aber keine Histomonaden. Auch dies zeigt eindeutig auf, dass es sich bei Protozoenkulturen meist um Co-Infektion bzw. Co-Isolierung handelt.

Bishop (1938) beschreibt auch die Isolierung von *Trichomonas, Chilomastix* und *Blastocystis* aus dem Zäkum eines Huhns.

In Norton et al. (1999) wird die Präsenz von *Histomonas meleagridis* und *Tetratrichomonas gallinarum* nach Infektion mit dem Nematoden *Ascaridia dissimilis* beschrieben.

Dies zeigt auf, dass klonale Protozoenkulturen bis dato nicht bekannt waren und ebenso nicht zur Verfügung gestellt werden konnten.

In der EP 600 396 A2 sind Verfahren und Mittel zur Prävention bzw. zur Behandlung von intestinalen Protozoeninfektionen, insbesondere von *Giardia lamblia,* geoffenbart. Dabei werden zunächst Gardia *lamblia* Stämme unterschiedlichster Herkunft kultiviert und geerntet, wobei die gewonnenen Protonen mittels Ultraschall zerstört werden, um nach weiterer Konzentrierung und Behandlung ein entsprechendes Vakkzin zu gewinnen.

In der GB 902 760 werden Verfahren zur Herstellung von attenuierten Endoparasiten beschrieben, bei denen ultraviolette Strahlung dazu verwendet wird, diese zu attenuieren.

FR 2 853 550 betrifft einen Nahrungsmittelzusatz zur Verabreichung an Tieren, der Zimtsäure, Aldehyd und Thiosulfat umfasst. Dabei wird aufgezeigt, dass eine Mischung enthaltend diese Wirkstoffe in der Lage ist, Protozoen wie *fetratrichomonas gallinarum* und *Histomonas meleagridis* effektiv zu bekämpfen.

Tan Kevin et al. (Experimental Parasitology 96(1) (2000): 9-15) betrifft die Kultivierung von *Blastocystis* hominis Kulturen auf festem Agar.

Die US 5 824 537 betrifft unter anderem ein Verfahren zur in vitro-Klonierung von Parasiten des Genus Babesia, wobei einzelne Parasiten-Zellen von einer Suspension von infizierten Erythrozyten gewonnen werden, wobei diese einzelnen Zellen schließlich zur Herstellung einer Kultur verwendet werden können.

Clark et al. (Clinical Microbiology Reviews 15(3) (2002): 329-341 ist ein Übersichtsartikel über Verfahren zur Kultivierung von Protozoen, wobei unter anderem die Züchtung von *Blastocystin hominis* beschrieben wird.

Bedingt durch die genetische Vielfalt, aber insbesondere durch das gleichzeitige Vorkommen mehrerer Protozoen in einem Tier, sind exakte diagnostische Methoden unabdingbar. Dies wird dadurch verstärkt, dass offensichtlich *Tetratrichomonas gallina*rum und *Histomonas meleagridis* ähnliche Symptome und morphologische Veränderungen hervorrufen können (Tyzzer, 1920; Allen, 1941).

Die Diagnostik der Histomoniasis wird besonders dadurch erschwert, dass der Parasit in der Außenwelt rasch abstirbt und dadurch nicht mehr nachzuweisen ist. Es besteht zwar die Möglichkeit der Kultivierung des Erregers aus körperwarmen Tieren, jedoch sind besondere Bedingungen notwendig, um das Überleben des Trophozoiten zu gewährleisten (Dwyer, 1970; McDougald and Galloway, 1973). Der direkte Erregernachweis kann somit nur mittels Histologie durchgeführt werden. Hierfür sind mehrere Methoden beschrieben, insbesondere die Perjodsäure-Schiff-Reaktion (PAS-Reaktion), mittels derer der im Gewebe unbegeißelte Erreger nachgewiesen werden kann (Kemp und REID, 1966). Allerdings ist diese Methode arbeits- und zeitaufwendig, was die Notwendigkeit nach Alternativen unterstreicht. Erschwert wird die Histologie auch dadurch, dass der Erreger von Makrophagen und Pilzzellen nicht leicht differenziert werden kann, was den Bedarf nach besseren Diagnosemöglichkeiten zusätzlich unterstreicht. Esquenet et al. (2003) beschreiben den zytologischen Nachweis mittels Direktfärbung von Abklatschpräparaten.

Molekulare diagnostische Nachweismethoden zum sensitiven und spezifischen Nachweis der drei Erreger sind bisher nicht vorhanden.

Aufgabe der Erfindung ist es, klonale Kulturen von *Histomonas meleagridis, Tetratrichomonas gallinarum* und *Blastocystis* sp. zu etablieren und solchermaßen etablierte Kulturen *in vitro* und *in vivo* anzuwenden, um die Nachteile des Standes der Technik, insbesondere die Inhomogenität der bisher bekannten Protozoenkulturen aus Geflügel, zu überwinden. Darüber hinaus ist es eine Aufgabe, spezifische Diagnostika zu entwickeln, damit ein sicherer Erregernachweis und eine Abgrenzung zwischen den Erregern möglich ist.

Demgemäß wird mit der vorliegenden Erfindung eine klonale Protozoenkultur umfassend Protozoenzellen einer einzigen Protozoenspezies zur Verfügung gestellt, welche dadurch gekennzeichnet ist, dass die Protozoenspezies ausgewählt ist aus der Gruppe bestehend aus *Histomonas meleagridis, Tetratrichomonas gallinarum* und *Blastocystis sp.,* wobei die Kultur symbiotische Bakterien oder deren Lysat enthält.

Gemäß der vorliegenden Erfindung wird als "klonale Protozoenkultur" eine Kultur von Protozoenzellen bezeichnet, die von einer einzelnen Protozoenzelle, die entweder *Histomonas meleagridis, Tetratrichomonas gallinarum* oder *Blastocystis sp.* ist, abstammt und somit aus dieser gebildet wurde. Die Kultur besteht somit aus Protozoenzellen, die alle durch Vermehrung von einer einzigen Zelle abstammen. Die Protozoenzellen sind somit Kopien einer einzigen oben angeführten Ausgangszelle. In der klonalen Kultur können sich zwar andere Organismen, wie Bakterien, befinden, erfindungswesentlich ist aber, dass nur eine einzige Protozoenspezies ausgewählt aus der Gruppe bestehend aus *Histomonas meleagridis, Tetratrichomonas gallinarum* und *Blastocystis sp.* in der klonalen Kultur vorhanden ist, d.h. die Kultur enthält Protozoenzellen einer dieser Protozoenspezies, nicht aber Protozoenzellen der beiden jeweils anderen Protozoenspezien (z.B. *Histomonas meleagridis* nicht aber *Tetratrichomonas gallinarum* und *Blastocystis sp., Tetratrichomonas gallinarum* nicht aber *Histomonas meleagridis* und *Blastocystis sp*.).

Die Bereitstellung einer derartigen klonalen Kultur konnte bisher im Stand der Technik - trotz des seit mindestens 20 bis 30 Jahren bestehenden Bedürfnisses (siehe oben Dywer oder Stepkowski) - nicht erfolgen und ermöglicht nunmehr aber eine Vielzahl von Anwendungen, die von der Herstellung von Impfstoffen und Therapeutika gegen die hierin angeführten Protozoenspezien bis hin zur näheren Erforschung und Charakterisierung derselben in Geflügel parasitischen Protozoenspezien reichen.

Bei der Herstellung von erfindungsgemäßen klonalen Protozoenkulturen ist es einerseits entscheidend das Wachstum der Protozoenzellen durch Vorsehen entsprechender Umgebungsbedingungen (z.B. Temperatur, Anwesenheit von symbiotischen Bakterien, Kohlenstoffquellen) *in vitro* zu ermöglichen und andererseits die Protozoen zu vereinzeln. Die Vereinzelung erfolgt vorzugsweise durch Mikromanipulation, wobei nach dieser Vereinzelung die Protozoen in einem entsprechenden Wachstumsmedium (das Wachstumsmedium sollte dabei eine Kohlenhydratquelle, insbesondere eine Stärke, wie Reis- oder Maisstärke, und gegebenenfalls Salze, insbesondere Earle's Salze, Aminosäuren, insbesondere L-Glutamin, und Antibiotika/Antimykotika, insbesondere Penicillin, Streptomycin und Amphotericin, umfassen) vermehrt werden, um schließlich eine klonale Protozoenkultur zu ergeben.

Die erfindungsgemäße Protozoenkultur enthält symbiotische Bakterien und/oder deren Lysat.

Es ist bekannt, dass sich *Histomonas meleagridis, Tetratrichomonas gallinarum* und *Blastocystis sp.* in Anwesenheit von symbiotischen Bakterien oder deren Stoffwechselprodukte (z.B. in Form von Lysat) *in vitro* effizient vermehren lassen. Aus diesem Grund befinden sich vorzugsweise symbiotische Bakterien und/oder deren Lysat in der erfindungsgemäßen Protozoenkultur, da diese bei der Herstellung der Kulturen eingesetzt werden. Dabei werden vorzugsweise jene Bakterien (und/oder Lysate) verwendet, die sich auch *in vivo* in Symbiose mit den Protozoen befinden. Selbstverständlich ist es auch möglich, die Bakterien im Wesentlichen vollständig aus der Protozoenkultur zu entfernen (z.B. durch Zentrifugation oder Filtration), sofern die Protozoenzellen beispielsweise zur Untersuchung von Wachstumsbedingungen, zur Herstellung von Impfstoffen und Antikörpern verwendet werden.

Gemäß einer bevorzugten Ausführungsform sind die Bakterien Enterobakterien, insbesondere coliforme Bakterien, Staphylokokken, Streptokokken oder Mischungen davon.

Da *Histomonas meleagridis, Tetratrichomonas gallinarum* und-*Blastocystis sp.* vorzugsweise aus Kot gewonnen werden, setzt sich die Bakterienkultur vorzugsweise aus den darin befindlichen Bakterienspezien zusammen. Es wurde erfindungsgemäß festgestellt, dass im Kot von Geflügel neben Enterobakterien auch Staphylokokken und Streptokokken vorhanden sind, die einzeln oder in Kombination zur Kultivierung von Protozoenzellen eingesetzt werden können.

Ein weiterer Aspekt der vorliegenden Erfindung betrifft eine Impfstoffformulierung umfassend inaktivierte oder lebend klonale Protozoenzellen einer einzigen Protozoenspezies ausgewählt aus der Gruppe bestehend aus *Histomonas meleagridis, Tetratrichomonas gallinarum* und *Blastocystis sp.* oder einen Antikörper gerichtet gegen klonale Protozoenzellen einer einzigen Protozoenspezies ausgewählt aus der Gruppe bestehend aus *Histomonas meleagridis, Tetratrichomonas gallinarum* und *Blastocystis sp..*

Die Bereitstellung von klonalen Protozoenkulturen bzw. -zellen ermöglicht eine gezielte Impfstoffherstellung, die einerseits zur aktiven und andererseits zur passiven Immunisierung von Geflügel eingesetzt werden kann. Bei der Herstellung von aktiven Impfstoffen ist es notwendig ein möglichst homogenes Antigen zu haben. Im konkreten Fall ist es wünschenswert, eine klonale Protozoenkultur zur Verfügung zu stellen, die im Wesentlichen nur eine Protozoenspezies enthält, gegen die ein Tier geimpft werden soll. Die Verwendung der erfindungsgemäßen klonalen Kulturen ermöglicht eine entscheidend verbesserte und standardisierbare industrielle Herstellung dieses Impfstoffes, vor allem im Hinblick auf bessere Reproduzierbarkeit und geringere Chargen-Varianz. Beispielsweise sollte ein aktiver Impfstoff, der zur Immunisierung von Geflügel gegen *Histomonas meleagridis* eingesetzt wird, im Wesentlichen nur *Histomonas meleagridis* Zellen umfassen. Ist dies nicht der Fall, kann es unter Umständen dazu führen, dass ein Selektionsprozess eintritt, der notwendigerweise nicht die gewünschte Spezies bevorzugt. D.h. es ist dann durchaus möglich, dass das Geflügel nicht oder unzureichend gegen den gewünschten Erreger immunisiert wird sondern gegen einen oder mehrere andere in der Kultur befindliche Antigene (z.B. andere Protozoen). Bei passiven Impfstoffen hingegen ist es wichtig, dass größtenteils Antikörper verwendet werden, die spezifisch gegen die zu bekämpfenden Antigene (z.B. Protozoenzellen) wirken. Daher ist es entscheidend, dass bei der Herstellung derartiger Impfstoffe möglichst reine Ausgangskulturen verwendet werden. Hierzu eignen sich besonders die erfindungsgemäßen klonalen Protozoenkulturen.

Die Impfstoffformulierung umfasst vorzugsweise ein Adjuvans.

Um die Immunantwort gegen ein Antigen zu fördern, werden bei der Herstellung aktiver Impfstoffe Adjuvantien verwendet, wobei das Adjuvans vorzugsweise ausgewählt ist aus der Gruppe bestehend aus Freund's komplettes Adjuvans, Freund's inkomplettes Adjuvans, Aluminiumhydroxid, Bordetella pertussis, Saponin, Muramyl-Dipeptid, Ethylen-Vinyl-Acetat Copolymer, Öl, vorzugsweise ein pflanzliches Öl oder ein Mineralöl, insbesondere Erdnussöl oder Silikonöl, und Kombinationen davon sowie die in Singh et al. (Nat. Biotech 17 (1999), 1075-1081) beschriebenen Adjuvantien.

Geeignete Adjuvantien, die erfindungsgemäß verwendet werden können, sind dem Fachmann hinreichend bekannt (siehe z.B. O'Hagan, Derek T. (2000) "Vaccine Adjuvants: Preparation Methods and Research Protocols", Humana Press; US 5,885,589; EP 109 942).

Die klonalen Protozoenzellen werden vorzugsweise durch Passagieren, durch Strahlung, insbesondere durch UV- und radioaktive Strahlung, durch chemische Substanzen oder durch Viren attenuiert.

Um die Pathogenität der im Impfstoff eingesetzten Protozoenzellen zu reduzieren, können diese Zellen attenuiert werden. Eine Attenuierung kann erfindungsgemäß durch mehrere Methoden erfolgen. Es hat sich erfindungsgemäß herausgestellt, dass vor allem die oben angeführten Attenuierungsverfahren sich besonders gut eignen die Pathogenität der Protozoenzellen zu reduzieren. Insbesondere wird die Attenuierung durch mehrmaliges Passagieren (mindestens 10, vorzugsweise mindestens 50, noch mehr bevorzugt mindestens 100 Passagen) bevorzugt verwendet. Beim Passagieren werden die Protozoen zunächst in einem Kulturgefäß vermehrt und nach dem Erreichen einer bestimmten Zelldichte (z.B. nach 12 Stunden, vorzugsweise nach 24 Stunden, noch mehr bevorzugt nach 48 Stunden, Kultivierung) in einem Verhältnis von beispielsweise 1:10, 1:50, 1:20 oder 1:5 in ein neues Medium überführt.

Gemäß einer bevorzugten Ausführungsform sind die klonalen Protozoenzellen durch Hitze, Formalin, beta-Propiolaktonoder Kombinationen davon inaktiviert.

Alternativ bzw. in Ergänzung zur Attenuierung der Protozoenzellen können diese auch durch mehrere Verfahren inaktiviert werden. Durch die Inaktivierung der Zellen wird das Risiko einer Infektion des geimpften Tieres drastisch reduziert.

Die Impfstoffformulierung ist vorzugsweise zur intravenösen, subkutanen, intramuskulären, oralen oder intrakloakalen Verabreichung adaptiert.

Ein weiterer Aspekt der vorliegenden Erfindung betrifft ein Verfahren zur Herstellung einer klonalen Protozoenkultur umfassend Protozoenzellen einer einzigen Protozoenspezies ausgewählt aus der Gruppe bestehend aus *Histomonas meleagridis, Tetratrichomonas gallinarum* und *Blastocystis sp.* umfassend die Schritte:
a) Bereitstellen einer Ausgangsprobe umfassend Protozoenzellen mindestens einer Protozoenspezies ausgewählt aus der Gruppe bestehend aus *Histomonas meleagridis, Tetratrichomonas gallinarum* und *Blastocystis sp.,*
b) Kultivieren der Zellen der Ausgangsprobe in einem Kulturmedium umfassend eine Kohlenhydratquelle und Bakterien mindestens einer Bakterienspezies, die *in situ* symbiotisch mit den Protozoenzellen leben,
c) Vereinzeln der Protozoenzellen mittels Mikromanipulation,
d) Kultivieren der vereinzelten Protozoenzellen in einem Kulturmedium wie unter b) definiert und
e) gegebenenfalls Bestimmen der Reinheit der klonalen Protozoenkultur.

Die Ausgangsprobe ist vorzugsweise Kot oder Gewebe.

Da *Histomonas meleagridis, Tetratrichomonas gallinarum* und *Blastocystis sp.* im Darm von Geflügel einen Teil ihres Lebenszyklus verbringen, ist besonders Kot geeignet, um diese Protozoen zu isolieren und daraus klonale Protozoenkulturen herzustellen.

Gemäß einer bevorzugten Ausführungsform wird die Ausgangsprobe von einem Geflügel ausgewählt aus der Gruppe bestehend aus Pute, Huhn, Fasan, Pfau, Wachtel und Perlhuhn bereitgestellt.

*Histomonas meleagridis, Tetratrichomonas gallinarum* und *Blastocystis sp.* sind Parasiten, die vornehmlich die obigen Geflügelarten befallen. Daher werden die Protozoen vorzugsweise aus diesen Tieren isoliert.

Die Kohlenhydratquelle ist vorzugsweise Stärke, insbesondere Reis-, Mais- oder Kartoffelstärke.

Es hat sich herausgestellt, dass *Histomonas meleagridis, Tetratrichomonas gallinarum* und *Blastocystis sp.* vor allem Stärke als Kohlenstoffquelle bevorzugen und diese durch Aufnahme in das Innere der Zelle abzubauen vermögen.

Die Bakterien sind vorzugsweise Enterobakterien, insbesondere coliforme Bakterien, Staphylokokken, Streptokokken oder Mischungen davon.

Es hat sich erfindungsgemäß herausgestellt, dass *Histomonas meleagridis, Tetratrichomonas gallinarum* und *Blastocystis sp.* insbesondere durch Symbiose mit den oben angeführten Bakterien (oder deren Lysate) kultivierbar sind. Ohne die Anwesenheit dieser Bakterienkulturen, die vorzugsweise aus dem Kot von Geflügel isoliert werden, können die Protozoen *ex vivo* nicht überleben und nicht kultiviert werden. Daher ist es entscheidend, dass die Bakterien sowohl vor als auch nach der Vereinzelung bei jedem ex vivo-Kultivierungsschritt, sofern die Lebensfähigkeit der Protozoen erwünscht ist, anwesend sind.

Vorzugsweise enthält das Kulturmedium weiters Salze, insbesondere Earle's Salze, Aminosäuren, insbesondere L-Glutamin, und Antibiotika/Antimykotika, insbesondere Penicillin, Streptomycin und Amphotericin.

Gemäß einer bevorzugten Ausführungsform wird die Reinheit der Kultur mittels einer Nukleinsäureamplifkations- oder Hybridisierungstechnik oder mittels einer immunologischen Technik mit Antikörpern gerichtet gegen klonale Protozoenzellen einer einzigen Protozoenspezies ausgewählt aus der Gruppe bestehend aus *Histomonas meleagridis, Tetratrichomonas gallinarum* und *Blastocystis sp.* bestimmt.

Die Bestimmung der Reinheit dient vor allem dazu die Anwesenheit des zu isolierden Parasiten bzw. die Abwesenheit zusätzlicher nicht in der klonalen Protozoenkultur erwünschten Zellen anderer Protozoenspezies zu bestätigen. Zur Bestimmung der Reinheit können mehrere Methoden verwendet werden, wobei aber Nukleinsäureamplifikations- und Hybridisierungstechniken und immunologische Techniken bevorzugt sind. Die Kenntnis konservierter bzw. Spezies-spezifischer Nukleinsäureregionen innerhalb der Protozoen-Genome ermöglicht es spezifische Primer bzw. Sonden zu identifizieren (z.B. durch Sequenz-Überlagerungen), herzustellen und einzusetzen (z.B. PCR, Southern-Blot, In Situ Hybridisierung). Derartige Techniken sind dem Fachmann hinreichend bekannt (siehe z.B. "Current Protocols in Molecular Biology", Ed Fred M. Ausubel et al., Wiley Inc.). Aufgrund der Möglichkeit, mit Hilfe der vorliegenden Erfindung klonale Protozoenkulturen mit einer einzigen Protozoenspezies herzustellen, ist es des Weiteren möglich, spezifische Antikörper gegen diese Protozoenspezies herzustellen und hierin einzusetzen, um die Reinheit der Kulturen zu bestimmen (z.B. mittels ELISA, RIA).

Vorzugsweise werden die Oligonukleotide 5'-GAAAGCATCTATCAAGTGGAA-3' und 5'-GATCTTTTCAAATTAGCTTTAAA-3' zur Bestimmung von *Histomonas meleagridis,* 5'-GCAATTGTTTCTCCAGAAGTG-3' und 5'-GATGGCTCTCTTTGAGCTTG-3' zur Bestimmung von *Tetratrichomonas gallinarum,* 5'-TAACCGTAGTAATTCTAGGGC-3' und 5'-AACGTTAATATACGCTATTGG-3' zur Bestimmung von *Blastocystis sp.* verwendet.

Diese Oligonukleotide erwiesen sich als besonders geeignet die einzelnen Protozoenspezien zu bestimmen, da diese an jene Genomregionen, die für die ribosomale RNA (rRNA) codieren, die wiederum zur phylogenetischen Charakterisierung von Organismen herangezogen wird, binden. Die Oligonukleotide wurden aufgrund von Sequenz-Überlagerungen von öffentlich zugänglichen rRNA-Sequenzdaten *Histomonas meleagridis* (AF293d56), *Tetratrichomonas gallinarum* (AF124608) und *Blastocystis sp.* (AF408427) ermittelt.

Nach dem erfindungsgemäßen Verfahren ist eine klonale Protozoenkultur: erhältlich, welche Protozoenspezies ausgewählt ist aus der Gruppe bestehend aus *Histomonas meleagridis, Tetratrichomonas gallinarum und Blastocyctis sp..*

Ein weiterer Aspekt der vorliegenden Erfindung betrifft ein Verfahren zur Bestimmung des Einflusses von Substanzen oder Substanzgemischen auf Protozoenzellen einer Protozoenspezies ausgewählt aus der Gruppe bestehend aus *Histomonas meleagridis, Tetratrichomonas gallinarum* und *Blastocystis sp.* umfassend die Schritte:
- Bereitstellen einer erfindungsgemäßen klonalen Protozoenkultur,
- gegebenenfalls Einstellen der Zellzahl der Protozoenkultur,
- Inkontaktbringen und Kultivieren der klonalen Protozoenkultur mit einem erfindungsgemäßen Kulturmedium,
- Zugabe mindestens einer Substanz oder mindestens eines Substanzgemisches, die/das potentiell einen Einfluss auf Protozoenzellen ausüben kann,
- Bestimmen der Protozoenzellzahl in der Protozoenkultur,
- Vergleichen der Protozoenzellzahl und der Begleitflora vor und nach Zugabe der mindestens einen Substanz oder des mindestens einen Substanzgemisches.

Das Bereitstellen einer klonalen Protozoenkultur ermöglicht es des Weiteren ein Verfahren zur Verfügung zu stellen, das geeignet ist, den Einfluss von Substanzen bzw. Substanzgemischen auf das Wachstumsverhalten und auf die Überlebensfähigkeit spezifischer Protozoen zu untersuchen. Da es bis dato nicht möglich war klonale Protozoenkulturen, die nur eine einzige Protozoenspezies (*Histomonas meleagridis, Tetratrichomonas gallinarum* und *Blastocystis sp.*) umfasst, herzustellen, konnten auch keine Untersuchungen durchgeführt werden, mit denen gezeigt werden konnte inwieweit sich bestimmte Substanzen auf den Stoffwechsel einer konkreten Protozoenspezies auswirken. Ein solches Verfahren ist von entscheidender Bedeutung bei der Erforschung von neuen Geflügel-Therapeutika, die gegen *Histomonas meleagridis, Tetratrichomonas gallinarum* und *Blastocystis sp.* wirksam sind.

Es hat sich gezeigt, dass die Wirkung von Substanzen auf Protozoen am geeignetsten durch die Veränderung der Zellzahl bestimmt wird (siehe z.B. Zenner et al., 2003; Caillait et al., 2002). Dazu wird zunächst die Anzahl der in einer Kultur vorhandenen Protozoen bestimmt und gegebenfalls auf eine bestimmte Anzahl eingestellt. Anschließend wird die klonale Kultur in Anwesenheit von Substanzen, die das Wachstum der Protozoen potentiell stören könnten, kultiviert. Nach einer Inkubationszeit von mindestens 12 Stunden, vorzugsweise mindestens 24 Stunden, wird die Anzahl der Protozoen neuerlich bestimmt und mit der Ausgangsanzahl bzw. mit einer Negativ-Kontrolle (Kulturmedium ohne Zusatz von potentiellen Inhibitoren) verglichen. Eine Abnahme der Zellzahl gegenüber der Ausgangsprobe bzw. eine reduzierte Anzahl von Protozoen gegenüber der Negativ-Kontrolle zeigt, dass die Substanz einen negativen Einfluss auf die Protozoenspezies hat. Die Anzahl der Parasiten wird einfach Bestimmung der Zellzahl bestimmt (z.B. lebend-tot Färbung).

Ein weiterer Aspekt der vorliegenden Erfindung betrifft ein Verfahren zum Nachweis einer Protozoenspezies ausgewählt aus der Gruppe bestehend aus *Histomonas meleagridis, Tetratrichomonas gallinarum* und *Blastocystis sp.* in einer Probe umfassend die Schritte:
- Bereitstellen einer Probe,
- Inkontaktbringen der Probe mit zumindest einen für *Histomonas meleagridis, Tetratrichomonas gallinarum* oder *Blastocystis sp.* spezifischen Oligonukleotid oder einen Antikörper gerichtet gegen *Histomonas meleagridis, Tetratrichomonas gallinarum* oder *Blastocystis sp.,*
- gegebenenfalls Unterwerfen des Proben/Oligonukleotid-Gemisches einer Nukleinsäureamplifikationstechnik,
- Detektion eines Nukleinsäureamplifikationsprodukts, einer Oligonukleotide/Protozoenzellen-Bindung oder einer Protozoenzellen/Antikörper-Bindung.

Die Bereitstellung klonaler Protozoenkulturen ermöglicht es Mittel (z.B. Antikörper) zu etablieren, die dazu verwendet werden können, Protozoen spezifisch in einer Probe nachzuweisen. Vorzugsweise ist die Probe eine Gewebe-, Kot-, Kultur- oder Umweltprobe (z.B. eine Probe aus einem Geflügelstall oder -gehege) isoliert.

Vorzugsweise werden die Oligonukleotide 5 -GAAAGCATCTATCAAGTGGAA-3' und 5'-GATCTTTTCAAATTAGCTTTAAA-3' zur Bestimmung von *Histomonas meleagridis,* 5'-GCAATTGTTTCTCCAGAAGTG-3' und 5'-GATGGCTCTCTTTGAGCTTG-3' zur Bestimmung von *Tetratrichomonas gallinarum,* 5'-TAACCGTAGTAATTCTAGGGC-3' und 5 -AACGTTAATATACGCTATTGG-3' zur Bestimmung von *Blastocystis sp.* verwendet.

Die Oligonukleotide können sowohl als Primer/Primerpaar zur Amplifikation eines Genomabschnitts mittels z.B. PCR als auch als Hybridisierungssonden verwendet werden.

Ein weiterer Aspekt der vorliegenden Erfindung betrifft ein Set zur Detektion von *Histomonas meleagridis, Tetratrichomonas gallinarum* und *Blastocystis sp.* oder Antikörper gerichtet gegen einer dieser Zellen in einer Probe umfassend:
a) klonale Protozoenzellen einer einzigen Protozoenspezies ausgewählt aus der Gruppe bestehend aus *Histomonas meleagridis, Tetratrichomonas gallinarum* und *Blastocystis sp.* oder einen Antikörper gerichtet gegen Protozoenzellen einer einzigen Protozoenspezies ausgewählt aus der Gruppe bestehend aus *Histomonas meleagridis, Tetratrichomonas gallinarum* und *Blastocystis sp.* und
b) Mittel zur Detektion der Protozoenzellen/Antikörper-Bindung.

Das erfindungsgemäße Set kann dazu verwendet werden *Histomonas meleagridis, Tetratrichomonas gallinarum* und *Blastocystis sp.* oder aber auch das Vorhandensein von Antikörpern gegen diese Protozoenzellen in einer Probe nachzuweisen. Der Nachweis von Antikörpern in einer Probe (Protozoenzellen werden bereitgestellt und mit einer Probe inkubiert) dient beispielsweise dazu den Immunisierungsverlauf eines Geflügels zu beobachten oder lediglich um festzustellen, ob das Geflügel bereits einen ausreichenden schützenden Antikörpertiter gegen *Histomonas meleagridis, Tetratrichomonas gallinarum* oder *Blastocystis sp.* aufweist.

Vorzugsweise sind die klonalen Protozoenzellen oder die Antikörper an einen festen Träger immobilisiert.

Es ist von Vorteil, wenn entweder die Protozoenzellen oder die Antikörper gegen diese Protozoenzellen auf einem festen Träger gebunden sind.

Gemäß einer bevorzugten Ausführungsform sind die Mittel zur Detektion konjugierte, vorzugsweise markierte Antikörper, insbesondere radioaktiv, Enzym- oder Fluoreszenz-markierte Antikörper.

Verfahren zur Detektion von Antikörper/Ligand-Bindungen sind dem Fachmann hinreichend bekannt (siehe z.B. "Immunochemical Protocols", 3rd Edition (2005), ed. Burns, Robert, Humana Press).

Ein weiterer Aspekt der vorliegenden Erfindung betrifft ein Futtermittel oder einen Futtermittelzusatzstoff umfassend eine erfindungsgemäße Impfstoffformulierung.

Ein Futtermittel, welches zur Impfung von Geflügel gegen *Histomonas meleagridis, Tetratrichomonas gallinarum* oder *Blastocystis sp.* führt, hat sich als besonders vorteilhaft erwiesen, da dadurch die Impfung der Tiere einfach und effizient gestaltet werden kann.

Die vorliegende Erfindung wird weiters durch die nachfolgenden Figuren und Beispiele beschrieben, ohne jedoch auf diese beschränkt zu sein.

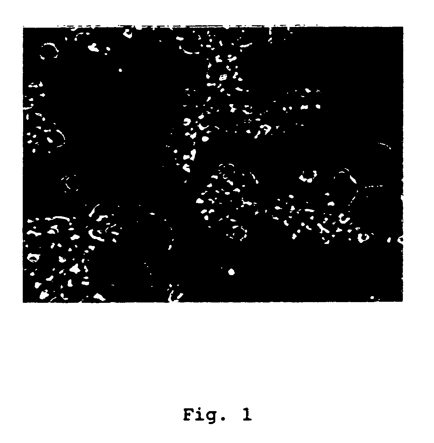

Figur 1 zeigt die lichtmikroskopische Darstellung (x 400; Strich: 10 µm) der Ausgangskultur A mit *Histomonas meleagridis (a), Tetratrichomonas gallinarum (b) and Blastocystis* sp (c).

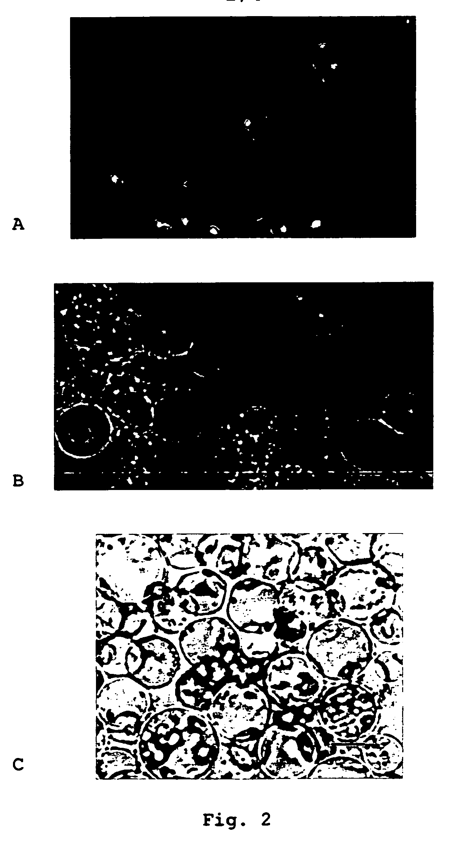

**Figur 2** zeigt klonale Kulturen von: *Histomonas meleagridis* **(A);** *Tetratrichomonas gallinarum* **(B)** and *Blastocystis* sp. **(C)** nach der Mikromanipulation.

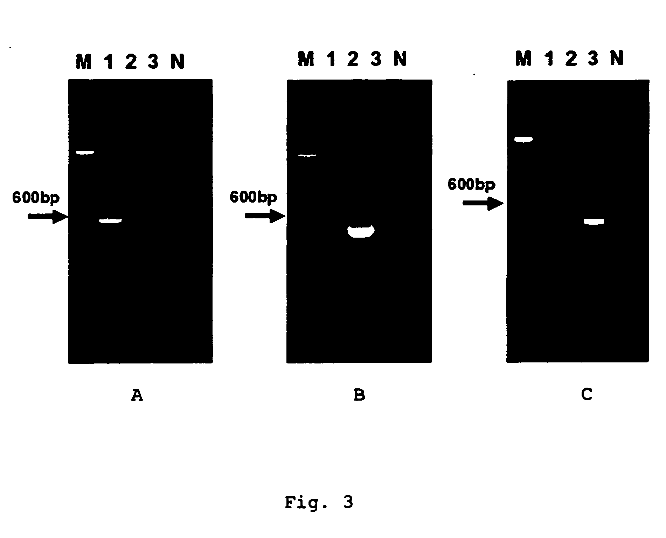

*Figur 3* *zeigt die Spezifität der entwickelten PCR-Assays. In keinem der Bilder sind Kreuzreaktionen, obwohl jeweils DNA Extrakte von 10⁵ Mikroorganismen von Histomonas meleagridis (Hm),Tetratrichomonas gallinarum (Tg) and Blastocystis* sp. *(B1)* für diese Untersuchung benutzt wurden. M: Molekulargewichtsmarker (100-bp ladder), 1: *Histomonas meleagridis, 2: Tetratrichomonas gallinarum 3: Blastocystis sp.;* N: Negativ Kontrolle. Verwendete Primer: A: Hmf / Hmr (Länge des PCR Produktes: 574 bp); B: Tgf / Tgr (527bp), C: Blf / Blr (457 bp). Oligonukleotide entsprechend der Tabelle 4.

### BEISPIELE:

### Beispiel 1: Herstellung klonaler Kulturen von Histomonas meleagridis, Tetratrichomonas gallinarum und Blastocystis sp..

Ausgangspunkt für die Arbeiten waren 2 Mischkulturen (A und B), die aus dem Kot von Puten angelegt wurden.

Die erste Kultur stammte aus einer 10 Wochen alten Pute, aus einem Bestand mit 5000 Tieren, wovon 40% starben und der Rest getötet wurde (Hess et al., 2004). Etwa 1 g des Blinddarmkots zusammen mit etwas Schleimhaut wurden in 9 ml Medium 199 + Earle's Salzen + L-Glutamine + 25 mM HEPES + L - Amino acids (Gibco™, Invitrogen) überführt. Zusätzlich wurden 11 mg Reisstärke (Sigma Aldrich), 10% fötales Kälberserum (Gibco™, Invitrogen), Antibiotika (200 IE Penicillin and 200 µg Streptomycin per ml Medium) und ein Antimykotikum (2,5 µg Amphotericin B/ml Medium) hinzugegeben. Dieses Medium wurde in allen nachfolgenden Untersuchungen als Standardmedium benutzt. Passagen wurden jeden zweiten Tag durchgeführt, wobei 1 ml des Mediums in ein neues steriles 50 ml Röhrchen (Sarstedt) überführt wurden, welches wiederum 9 ml des Standardmediums enthielt. Bei Passage 117 wurde die Kultur für die Mikromanipulation verwendet.

Die Ursprungskultur B wurde ähnlich wie Kultur A hergestellt. Allerdings wurde als Ausgangsmaterial Blinddarmmaterial von einer Bronzepute von einem Hobbybetrieb, in dem 2 von 6 Tieren starben, als Ausgangsmaterial verwendet. Für die Mikromanipulation wurde die Kultur B nach Passage 7 verwendet.

Um einzelne Zellen aus den Vorläuferkulturen A und B zu isolieren, wurde die Mikromanipulation verwendet, wie es schon für andere Protozoen beschrieben ist. So hat Farri et al. (Transactions of the Royal Society of Tropical Medicine and Hygiene 72(2) (1978): 205-206) diese Methode benutzt, um *Entamoeba histolytica* zu klonieren. Odula et al. (Experimental Parasitology 66(1988): 86-95) benutzten die Methode um den Erreger der Malaria, *Plasmodium* falciparum zu klonieren. Letztlich konnten Bushek et al. (J. Eucaryotic Microbiol. 47(2000): 164-166) klonale Kulturen des für Austern pathogenen Mikroorganismus Perkinsus marinus etablieren.

Alle Arbeiten nutzen die Eigenschaft der Mikromanipulation, dass einzelne Zellen manipuliert und somit separiert werden können.

Die Mikromanipulation wurde im Konkreten wie folgt durchgeführt:

Die beiden Ursprungskulturen (A und B) aus dem Kot von Puten wurden 1:100 verdünnt und mit einer Kapillare (1,0 mm äußerer Durchmesser, Hilgenberg, Deutschland), die auf einem Puller aufgebracht war (P97, Sutter, USA) und auf einen äußeren Durchmesser von 25 µm (Microforge deFonbrune, Bachofer, Deutschland) gezogen wurde. Parasiten wurden in 50 µl Kulturmedium gebracht und in ein Eppendorf-Röhrchen transferiert. Der gesamte Ablauf wurde mit einem Narishige Micromanipulator (Narishige, Japan) und einem Mikroskop (Diaphot 300, Nikon, Austria) unter 400-facher Vergrößerung durchgeführt. Zur Überwachung der Isolierung wurde eine CCD Kamera und ein Monitor (Sony, Japan), welche mit dem Mikroskop verbunden waren, eingesetzt. Nach der Isolierung wurden die Mikroorganismen bei 40°C bis zu 4 Tage in Eppendorf-Röhrchen bebrütet. Lichtmikroskopisch wurde das Wachstum am 2., 3. und 4. Tag überprüft. Jede positive oder verdächtige Kolonie wurde in 9,7 ml Standard Medium in ein 50 ml Plastikröhrchen (Sarstedt) überführt.

Entscheidend für die Kultivierung ist die Verwendung eines entsprechenden Wachstumssubstrates ("Seedermedium"). Dies ist notwendig, da zumindest Histomonas meleagridis, in Abwesenheit von Bakterien und einer Kohlenhydratquelle, insbesondere Stärke, wie Reisstärke, somit axenisch, nicht wächst. Um ein entsprechendes Wachstumssubstrat herzustellen, wurde die bakterielle Begleitflora der jeweiligen Ursprungskultur bestimmt. Zu diesem Zweck wurden ca. 100 µl der Ursprungskolonie auf kommerziellen Columbia (+5% blood), McConkey, Salmonella Detection and Identification Media (SMID) und Sabouraud Agar Platten (alle: bioMerieux, Marcy l'Etoile, France) ausgestrichen. Die Identifizierung der Bakterienflora erfolgte mit dem API-20 E Microsystem (bio Merieux, Marcy l'Etoile, Frankreich). Als hauptsächliche bakterielle Begleitflora wurden E.coli, Staphylokokken und Streptokokken identifiziert. Mit einer Impföse wurde ein Teil der Bakterien von der Columbia Blutagarplatte abgenommen und in das Anzuchtmedium vor der Mikromanipulation gegeben.

Um eine weitere Identifikation der Erreger durchzuführen, wurden Teile der ribosomalen RNA sequenziert und in eine öffentlich zugängliche Sequenzdatenbank eingegeben (Zugriffsnummern: AJ920322 - AJ920324).

### Beispiel 2: Bestimmung des Wachstumsverhaltens der klonalen Kulturen.

Nach der Isolierung wurden die Parasiten für je 3-4 Tage im Eppendorf-Röhrchen gehalten, bis sie in ein 50 ml Röhrchen, welches wiederum 9 ml Medium enthielt, überführt wurden. Anschließend wurden nach 3 - 4 Tagen 1 ml der Kultur in ein neues Röhrchen überführt, in welchem 9 ml des Standardmediums vorgelegt waren, mithin ein identisches Vorgehen wie bei den Ursprungskulturen A und B. Die weitere Kultivierung erfolgte dann analog dem Verfahren der Ausgangskulturen. Zu verschiedenen Zeitpunkten wurden Zählungen durchgeführt, um die Wachstumsrate der Einzeller zu bestimmen. Dabei wurde mittels Trypanblau (0,4%) eine Lebend- Totfärbung durchgeführt (Tabelle 1).

**Tabelle 1. Um das Wachstumsverhalten der klonierten Erreger zu überprüfen, wurde zu unterschiedlichen Zeitpunkten ein Wachstumsprofil erstellt, wie es in der folgenden Tabelle (1) dargestellt ist.**

| ***Organismus*** | ***Passagenzahl*** | ****Tag 0*** | **#24h** | ***48h*** | ***72h*** | ***96h*** |
|---|---|---|---|---|---|---|
| *Histomonas meleagridis* | 62 | 1,0 x 10⁴ | 2,75 x 10⁵ | 3,30 x 10⁵ | 7,45 x 10⁵ | 3,25 x 10⁵ |
| *Tetratrichomonas gallinarum* | 114 | 1,0 x 10⁴ | 7,5 x 10⁴ | 8,050 x 10⁶ | 8,450 x 10⁶ | 4,650 x 10⁶ |
| *Blastocystis* sp. | 74 | 6,4 x 10⁴ | 1,91 x 10⁶ | 7,275 x 10⁶ | 3,675 x 10⁶ | 4,0 x 10⁵ |

| | | | | | | |
|---|---|---|---|---|---|---|
| *Anzahl lebender Protozoen in 1 ml Medium, welche in 9 ml frisches Medium überführt wurden. "Anzahl der Protozoen/ml zu verschiedenen Zeitpunkten nach der Inkubation | | | | | | |

### Beispiel 3: Empfindlichkeit der Kulturen für das Standardpräparat Dimetridazol, als Referenz für die Testung von Substanzen in vitro.

Um die Möglichkeit der Substanztestung zu überprüfen, wurde die klonale Histomonas Kultur mit dem Imidazolderivat Dimetridazol versetzt.

Insgesamt drei 50ml Röhrchen der klonierten Histomonas Kultur wurden am 3. Tag der Anzucht bei 380 x g zentrifugiert. Anschließend wurde der Überstand abgenommen und verworfen. Die Pellets der 3 Röhrchen wurden gepoolt und die Histomonaden gezählt. Anschließend wurde mittels Zellkulturmedium eine Konzentration von 100.000/ml eingestellt. Jeweils 1 ml Histomonadenkultur wurde in ein Eppendorf-Röhrchen überführt und zusammen mit 0,4 mg Dimetridazol bei 40°C inkubiert. In 24h Abständen wird eine Zählung der Parasiten durchgeführt, um die Wirkung einer Testsubstanz zu untersuchen, wie in Tabelle 2 beispielhaft dargestellt wird.

**Tabelle 2: Wachstumsverlauf des klonierten Erregers Histomonas meleagridis, als Modell für die Substanztestung in vitro**

| | ^{a}Zeitpunkt 0 | ^{b}24h | 48h |
|---|---|---|---|
| Zusatz von 0,4mg Dimetridazol | 100.000 | ^{c} -- | -- |
| Kontrolle (ohne Zusatz) | 100.000 | 570.000 | 605.000 |

| | | | |
|---|---|---|---|
| ^{a}Anzahl lebender Protozoen in 1 ml Medium ^{b}Anzahl der Protozoen/ml zu verschiedenen Zeitpunkten nach der Inkubation ^{c} keine lebende Parasiten vorhanden | | | |

Das Beispiel zeigt deutlich die Effizienz des Wirkstoffes Dimetridazol.

### Beispiel 4: Reproduktion der Histomoniasis oder Schwarzkopfkrankheit in 14 Tage alten Puten, als Grundlage für die Etablierung eines in vivo Modells, geeignet für die Testung von Substanzen in vivo.

Um die Infektiosität der Kulturen zu überprüfen, wurde ein Tierversuch durchgeführt, wobei 14 Tage alte Puten intrakloakal infiziert wurden.

Kommerzielle Puten (18 Tiere) wurden als Eintagsküken eingestallt und mit Standardfutter versorgt. Im Alter von 14 Tagen wurden die 18 Tiere aufgeteilt dergestalt, dass 14 Tiere in einem Raum verblieben und 4 Tiere in einen weiteren Raum verbracht wurden. Von den 14 Tieren wurden 10 Tiere via Kloake mit je 200.000 Histomonaden (Passagenzahl: 7x bis zur Klonierung; 7x nach der Klonierung) infiziert. Die Tiere wurden täglich klinisch untersucht und sämtliche Auffälligkeiten (insbesondere Durchfälle) notiert. Tabelle 2 gibt eine Übersicht über den Verlauf der Erkrankung bei den infizierten und den Kontakttieren.

**Tabelle 3: Durchfallgeschehen und Krankheitsverlauf der Histomoniasis in 14 Tage alten Puten.**

| | **Kontrolltiere** | | | | | **Infizierte Tiere** | | | | | | | | | | **Kontakttiere** | | | |
|---|---|---|---|---|---|---|---|---|---|---|---|---|---|---|---|---|---|---|---|
| *Tag*/ *Tier* | *1* | *2* | *3* | *4* | | *5* | *6* | *7* | *8* | *9* | *10* | *11* | *12* | *13* | *14* | *15* | *16* | *17* | *18* |
| 6 | -^{a} | - | - | - | | - | - | - | - | - | - | - | - | - | - | - | - | - | - |
| 7 | - | - | - | - | | - | - | - | - | x | - | - | - | - | - | - | - | - | - |
| 8 | - | - | - | - | | - | - | - | - | - | x | - | - | - | x | - | - | - | - |
| 9 | - | - | - | - | | x^{b} | - | - | x | x | x | - | - | x | - | - | - | - | - |
| 10 | - | - | - | - | | x | x | x | - | x | x | x | - | x | - | - | x | - | x |
| 11 | - | - | - | - | | †^{c} | † | x | † | † | † | x | † | † | - | - | x | † | - |
| 12 | - | - | - | - | | | | † | | | | x | | | † | x | x | | - |
| 13 | - | - | - | - | | | | | | | | † | | | | † | - | | x |
| 14 | Tiere getötet | | | | | | | | | | | | | | | | † | | † |

| | | | | | | | | | | | | | | | | | | | |
|---|---|---|---|---|---|---|---|---|---|---|---|---|---|---|---|---|---|---|---|
| ^{a} -: kein Durchfall ^{b} x: typisch safranfarbener Durchfall ^{c} †: Tier verstorben | | | | | | | | | | | | | | | | | | | |

Die Tatsache, dass sämtliche Tiere (infizierte Tiere und Kontakttiere) zwischen dem 11. und 14. Tag nach der Infektion versterben, unterstreicht die Effizienz des etablierten Tiermodells.

### Beispiel 5: Etablierung molekularer Diagnostikmethoden zum spezifischen Nachweis von Histomonas meleagridis, Tetratrichomonas gallinarum und Blastocystis sp..

Die klonalen Kulturen wurden verwendet, um molekulare Nachweismethoden zu etablieren, mit der Eigenschaft den jeweiligen Erreger spezifisch nachzuweisen.

Zu diesem Zweck wurden Oligonukleotide konstruiert, mit deren Hilfe spezifisch der jeweilige Erreger nachgewiesen werden kann.

Basierend auf den genomischen Unterschieden im Bereich der ribosomalen RNA wurden Oligonukleotide konstruiert, die den spezifischen Nachweis des jeweiligen Erregers mittels Hybridisierung oder Amplifikation des Genomfragmentes (PCR) ermöglichen.

**Tabelle 4: Oligonukleotide zum spezifischen Nachweis von Histomonas meleagridis, Tetratrichomans gallinarum und Blastocystis sp..**

| ***Primer*** | ***Sequenz*** | ***Nukleotid Position^{a,b,c}*** | **Amplifikat** |
|---|---|---|---|
| Hmf | 5'- GAA AGC ATC TAT CAA GTG GAA -3' | 768 - 788 bp^{a} | 574 bp |
| Hmr | 5'- GAT CTT TTC AAA TTA GCT TTA AA -3' | 1342 - 1320 bp^{a} | |
| Tgf | 5'- GCA ATT GTT TCT CCA GAA GTG - 3' | 152 - 171 bp^{b} | 527 bp |
| Tgr | 5'- GAT GGC TCT CTT TGA GCT TG - 3' | 660 - 679 bp^{b} | |
| Blf | 5'- TAA CCG TAG TAA TTC TAG GGC -3' | 120 - 140 bp^{c} | 457 bp |
| Blr | 5'- AAC GTT AAT ATA CGC TAT TGG -3' | 557 - 577 bp^{c} | |

| | | | |
|---|---|---|---|
| ^{a}: nach Accession number: AF293056 ^{b}: nach Accession number: AF124608 ^{c}: nach Accession number: AF408427 | | | |

### Literatur:

Allen, E.A., (1941) Am. J. Vet. Res., 2:214-217.
Bayon and Bishop, (1937) Nature, 139:370-371.
Bishop A. (1938) Parasitology, 30: 181-194.
Burkot, T.R., et al. (1984) Trans. R. Soc. Trop. Med. Hyg. 78:339-341.
Callait, M.P., et al. (2002) Poult Sci. 81:1122-7.
Cepicka, I., et al. (2005) Vet. Parasitol. 128:11-21.
Dwyer, D.M., (1970) J. Parasitol. 56:191-192.
Esquenet, C., et al. (2003) Avian Pathol. 32:303-306.
Gerbod, D., et al. (2001) J. Eukaryot. Microbiol. 48:498-504.
Goedbloed and Bool, (1962) Avian Dis., 6:302-315.
Hafez, H. M., et al. (2001) Tierärztl. Praxis 3:168.
Hess, M., et al. (2004) 5th International Symposium on Turkey Diseases, Berlin, 16.-19.06.2004, 254-257.
Hu, J., et al. (2004) Avian Dis. 48:746-750.
Hu, J.H., McDougald, L.R., (2003) Avian Dis. 47:489-492.
Kemp, R.L., Reid, W.M., (1966) Avian Dis. 10:357-363.
Kulda et al. (1974) Acta Vet. Brno, 43:53-64.
Landmann, W.J.M., et al. (2004) 5th International Symposium on Turkey Diseases, Berlin, 16.-19.06.2004, 258-268.
LeBras, J., et al. (1983) Exp. Parasitol. 56:9-14.
McDougald, L.R., Galloway, R.B., (1973) Avian Dis. 17:847-850.
McDougald, L.R., (2003). Other protozoan diseases of the intestinal tract - Histomoniasis (Blackhead). In Saif, Y.M., Barnes, H.J., Glisson, J.R., Fadly, A.M., McDougald, L.R., Swayne, D.E., (Ed.), Diseases of Poultry, Iowa State Press, Ames, Iowa p 1001-1008.
Noel, C., et al. (2003) Mol. Biochem. Parasitol. 126:119-123.
Noel, C., et al. (2005) J. Clin. Microbiol. 43:348-355.
Norton et al. (1999) Avian Diseases, 43:342-348.
Rosario, V., (1981) Science 212:1037-1038.
Smith, T., (1895). An infectious disease among turkeys caused by Protozoa (infectious enterohepatitis). US Dept. Agric. Bur. Anim. Indust., Bull. No. 8, 7.
Stepkowski, S., Klimont, S., (1979). Obserwacje nad hodowlg in vitro Histomonas meleagridis (Smith, 1895), Med. Wet. 8:502-505.
Trager, W., et al. (1981) Proc. Natl. Acad. Sci. 78:6527-6530.
Tyzzer, E.E., (1920) J. Parasitol. 6:124-130.
Zenner, L., et al. (2003) Parasite. 10:153-7.

## Patentansprüche

1. Klonale Protozoenkultur umfassend Protozoenzellen einer einzigen Protozoenspezies, wobei die Protozoenspezies ausgewählt ist aus der Gruppe bestehend aus *Histomonas meleagridis, Tetratrichomonas gallinarum* und *Blastocystis sp.,* **dadurch gekennzeichnet, dass** die Kultur symbiotische Bakterien oder deren Lysat enthält.

2. Protozoenkultur nach Anspruch 1, **dadurch gekennzeichnet, dass** die Bakterien Enterobakterien, insbesondere coliforme Bakterien, Staphylokokken, Streptokokken oder Mischungen davon sind.

3. Impfstoffformulierung umfassend inaktivierte oder lebend klonale Protozoenzellen einer einzigen Protozoenspezies ausgewählt aus der Gruppe bestehend aus *Histomonas meleagridis, Tetratrichomonas gallinarum* und *Blastocystis sp.* oder einen Antikörper gerichtet gegen klonale Protozoenzellen einer einzigen Protozoenspezies ausgewählt aus der Gruppe bestehend aus *Histomonas meleagridis, Tetratrichomonas gallinarum* und *Blastocystis sp..*

4. Impfstoffformulierung nach Anspruch 3, **dadurch gekennzeichnet, dass** der Impfstoff ein Adjuvans umfasst, welches vorzugsweise ausgewählt ist aus der Gruppe bestehend aus Freund's komplettes Adjuvans, Freund's inkomplettes Adjuvans, Aluminiumhydroxid, Bordetella pertussis, Saponin, Muramyl-Dipeptid, Ethylen-Vinyl-Acetat Copolymer, Öl, vorzugsweise ein pflanzliches Öl oder ein Mineralöl, insbesondere Erdnussöl oder Silikonöl, und Kombinationen davon.

5. Impfstoffformulierung nach Anspruch 3 oder 4, **dadurch gekennzeichnet, dass** die klonalen Protozoenzellen durch Passagieren, durch Strahlung, insbesondere durch UV- und radioaktive Strahlung, durch chemische Substanzen oder durch Viren oder durch Hitze, Formalin, beta-Propiolakton oder Kombinationen davon inaktiviert sind.

6. Impfstoffformulierung nach einem der Ansprüche 3 bis 5, **dadurch gekennzeichnet, dass** der Impfstoff zur intravenösen, subkutanen, intramuskulären, oralen oder intrakloakalen Verabreichung adaptiert ist.

7. Verfahren zur Herstellung einer klonalen Protozoenkultur umfassend Protozoenzellen einer einzigen Protozoenspezies ausgewählt aus der Gruppe bestehend aus *Histomonas meleagridis, Tetratrichomonas gallinarum* und *Blastocystis sp.* umfassend die Schritte:
a) Bereitstellen einer Ausgangsprobe umfassend Protozoenzellen mindestens einer Protozoenspezies ausgewählt aus der Gruppe bestehend aus *Histomonas meleagridis, Tetratrichomonas gallinarum* und *Blastocystis sp.,*
b) Kultivieren der Zellen der Ausgangsprobe in einem Kulturmedium umfassend eine Kohlenhydratquelle und Bakterien mindestens einer Bakterienspezies, die *in situ* symbiotisch mit den Protozoenzellen leben, oder Lysat davon,
c) Vereinzeln der Protozoenzellen mittels Mikromanipulation,
d) Kultivieren der vereinzelten Protozoenzellen in einem Kulturmedium wie unter b) definiert und
e) gegebenenfalls Bestimmen der Reinheit der klonalen Protozoenkultur.

8. Verfahren nach Anspruch 7, **dadurch gekennzeichnet, dass** die Ausgangsprobe eine Kot-, Gewebe-, Kultur- oder Umweltprobe ist.

9. Verfahren nach Anspruch 7 oder 8, **dadurch gekennzeichnet, dass** die Ausgangsprobe von einem Geflügel ausgewählt aus der Gruppe bestehend aus Pute, Huhn, Fasan, Pfau, Wachtel und Perlhuhn bereitgestellt wird.

10. Verfahren nach einem der Ansprüche 7 bis 9, **dadurch gekennzeichnet, dass** die Kohlenhydratquelle Stärke, insbesondere Reis-, Mais- oder Kartoffelstärke, ist.

11. Verfahren nach einem der Ansprüche 7 bis 10, **dadurch gekennzeichnet, dass** die Bakterien Enterobakterien, insbesondere coliforme Bakterien, Staphylokokken, Streptokokken oder Mischungen davon sind.

12. Verfahren nach einem der Ansprüche 7 bis 11, **dadurch gekennzeichnet, dass** das Kulturmedium weiters Salze, insbesondere Earle's Salze, Aminosäuren, insbesondere L-Glutamin, und Antibiotika/Antimykotika, insbesondere Penicillin, Streptomycin und Amphotericin enthält.

13. Verfahren nach einem der Ansprüche 7 bis 12, **dadurch gekennzeichnet, dass** die Reinheit der Kultur mittels einer Nukleinsäureamplifkations- oder Hybridisierungstechnik oder mittels einer immunologischen Technik mit Antikörpern gerichtet gegen klonale Protozoenzellen einer einzigen Protozoenspezies ausgewählt aus der Gruppe bestehend aus *Histomonas meleagridis, Tetratrichomonas gallinarum* und *Blastocystis sp.* bestimmt wird.

14. Verfahren nach Anspruch 13, **dadurch gekennzeichnet, dass** die Oligonukleotide 5'-GAAAGCATCTATCAAGTGGAA-3' und 5'-GATCTTTTCAAATTAGCTTTAAA-3' zur Bestimmung von *Histomonas meleagridis,* 5'-GCAATTGTTTCTCCAGAAGTG-3' und 5'-GATGGCTCTCTTTGAGCTTG-3' zur Bestimmung von *Tetratrichomonas gallinarum,* 5'-TAACCGTAGTAATTCTAGGGC-3' und 5'-AACGTTAATATACGCTATTGG-3' zur Bestimmung von *Blastocystis sp.* verwendet werden.

15. Verfahren zur Bestimmung des Einflusses von Substanzen oder Substanzgemischen auf Protozoenzellen einer Protozoenspezies ausgewählt aus der Gruppe bestehend aus *Histomonas meleagridis, Tetratrichomonas gallinarum* und *Blastocystis sp.* umfassend die Schritte:
- Bereitstellen einer klonalen Protozoenkultur nach Anspruch 1 oder 2,
- gegebenenfalls Einstellen der Zellzahl der Protozoenkultur,
- Inkontaktbringen und Kultivieren der klonalen Protozoenkultur mit einem Kulturmedium wie in den Ansprüchen 7 und 10 bis 12 definiert,
- Zugabe mindestens einer Substanz oder mindestens eines Substanzgemisches, die/das potentiell einen Einfluss auf Protozoenzellen ausüben kann,
- Bestimmen der Protozoenzellzahl in der Protozoenkultur,
- Vergleichen der Protozoenzellzahl vor und nach Zugabe der mindestens einen Substanz oder des mindestens einen Substanzgemisches.

16. Verfahren zum Nachweis einer Protozoenspezies ausgewählt aus der Gruppe bestehend aus *Histomonas meleagridis, Tetratrichomonas gallinarum* und *Blastocystis sp.* in einer Probe umfassend die Schritte:
- Bereitstellen einer Probe,
- Inkontaktbringen der Probe mit zumindest einen für *Histomonas meleagridis, Tetratrichomonas gallinarum* oder *Blastocystis* sp. spezifischen Oligonukleotid oder einem Antikörper gerichtet gegen *Histomonas meleagridis, Tetratrichomonas gallinarum* oder *Blastocystis sp.,*
- gegebenenfalls Unterwerfen des Proben/Oligonukleotid-Gemisches einer Nukleinsäureamplifikationstechnik,
- Detektion eines Nukleinsäureamplifikationsprodukts, einer Oligonukleotid/Protozoenzellen-Bindung oder einer Protozoenzellen/Antikörper-Bindung.

17. Verfahren nach Anspruch 16, **dadurch gekennzeichnet, dass** die Oligonukleotide 5'-GAAAGCATCTATCAAGTGGAA-3' und 5'-GATCTTTTCAAATTAGCTTTAAA-3' zur Bestimmung von *Histomonas meleagridis,* 5'-GCAATTGTTTCTCCAGAAGTG-3' und 5'-GATGGCTCTCTTTGAGCTTG-3' zur Bestimmung von *Tetratrichomonas gallinarum,* 5'-TAACCGTAGTAATTCTAGGGC-3' und 5'-AACGTTAATATACGCTATTGG-3' zur Bestimmung von *Blastocystis sp.* verwendet werden.

18. Set zur Detektion von *Histomonas meleagridis, Tetratrichomonas gallinarum* und *Blastocystis sp.* oder Antikörper gerichtet gegen einer dieser Zellen in einer Probe umfassend:
a) klonale Protozoenzellen einer einzigen Protozoenspezies ausgewählt aus der Gruppe bestehend aus *Histomonas meleagridis, Tetratrichomonas gallinarum* und *Blastocystis sp.* oder einen Antikörper gerichtet gegen Protozoenzellen einer einzigen Protozoenspezies ausgewählt aus der Gruppe bestehend aus *Histomonas meleagridis, Tetratrichomonas gallinarum* und *Blastocystis sp.* und
b) Mittel zur Detektion der Protozoenzellen/Antikörper-Bindung.

19. Futtermittel oder Futtermittelzusatzstoff umfassend einen Impfstoff nach einem der Ansprüche 3 bis 6.

## Claims

1. A clonal protozoa culture comprising protozoa cells of a single protozoa species, wherein said protozoa species is selected from the group consisting of *Histomonas meleagridis, Tetratrichomonas gallinarum* and *Blastocystis sp.,* **characterized in that** said culture contains symbiotic bacteria or their lysate.

2. The protozoa culture according to claim 1, **characterized in that** said bacteria are enterobacteria, in particular coliform bacteria, staphylococci, streptococci, or mixtures thereof.

3. A vaccine formulation comprising inactivated or live clonal protozoa cells of a single protozoa species selected from the group consisting of *Histomonas meleagridis, Tetratrichomonas gallinarum* and *Blastocystis sp.,* or an antibody directed against clonal protozoa cells of a single protozoa species selected from the group consisting of *Histomonas meleagridis, Tetratrichomonas gallinarum* and *Blastocystis sp..*

4. The vaccine formulation according to claim 3, **characterized in that** said vaccine comprises an adjuvant, which preferably is selected from the group consisting of Freund's complete adjuvant, Freund's incomplete adjuvant, aluminum hydroxide, Bordetella pertussis, saponin, muramyl dipeptide, ethylene vinyl acetate copolymer, oil, preferably a vegetable oil or a mineral oil, in particular peanut oil or silicone oil, and combinations thereof.

5. The vaccine formulation according to claims 3 or 4, **characterized in that** said clonal protozoa cells are inactivated by passaging, by radiation, in particular by UV and radioactive radiation, by chemical substances or by viruses or heat, formalin, beta-propiolactone, or combinations thereof.

6. The vaccine formulation according to any of claims 3 to 5, **characterized in that** said vaccine is adapted for intravenous, subcutaneous, intramuscular, oral or intracloacal administration.

7. A method for producing a clonal protozoa culture comprising protozoa cells of a single protozoa species selected from the group consisting of *Histomonas meleagridis, Tetratrichomonas gallinarum* and *Blastocystis sp.,* comprising the steps of:
a) providing a starting sample comprising protozoa cells of at least one protozoa species selected from the group consisting of *Histomonas meleagridis, Tetratrichomonas gallinarum* and *Blastocystis sp.,*
b) cultivating the cells of said starting sample in a culture medium comprising a carbohydrate source and bacteria of at least one bacteria species, which *in situ* lives symbiotic with said protozoa cells, or lysate thereof,
c) individualizing said protozoa cells using micro-manipulation,
d) cultivating said isolated protozoa cells in a culture medium as defined under b), and
e) optionally, determining the purity of said clonal protozoa culture.

8. The method according to claim 7, **characterized in that** said starting sample is a faeces, tissue, culture or environmental sample.

9. The method according to claims 7 or 8, **characterized in that** said starting sample is provided by a poultry selected from the group consisting of turkey, chicken, pheasant, peacock, quail, and guinea fowl.

10. The method according to any of claims 7 to 9, **characterized in that** said carbohydrate source is starch, in particular rice, corn or potato starch.

11. The method according to any of claims 7 to 10, **characterized in that** said bacteria are enterobacteria, in particular coliform bacteria, staphylococci, streptococci, or mixtures thereof.

12. The method according to any of claims 7 to 11, **characterized in that** said culture medium further contains salts, in particular Earle's salts, amino acids, in particular L-glutamine, and antibiotics/antimycotics, in particular penicillin, streptomycin, and amphotericin.

13. The method according to any of claims 7 to 12, **characterized in that** the purity of said culture is determined using a nucleic acid amplification or hybridization technique or using an immunologic technique with antibodies directed against clonal protozoa cells of a single protozoa species selected from the group consisting of *Histomonas meleagridis, Tetratrichomonas gallinarum* and *Blastocystis sp..*

14. The method according to claim 13, **characterized in that** the oligonucleotides 5'-GAAAGCATCTATCAAGTGGAA-3' and 5'-GATCTTTTCAAATTAGCTTTAAA-3' are used for determining *Histomonas meleagridis,* 5'-GCAATTGTTTCTCCAGAAGTG-3' and 5'-GATGGCTCTCTTTGAGCTTG-3' for determining *Tetratrichomonas gallinarum,* and 5'-TAACCGTAGTAATTCTAGGGC-3' and 5'-AACGTTAATATACGCTATTGG-3' for determining *Blastocystis sp..*

15. A method for determining the influence of substances or mixtures of substances on protozoa cells of a protozoa species selected from the group consisting of *Histomonas meleagridis, Tetratrichomonas gallinarum* and *Blastocystis sp.,* comprising the steps of:
- providing a clonal protozoa culture according to claims 1 or 2,
- optionally, adjusting the cell count of said protozoa culture,
- contacting and cultivating said clonal protozoa culture with a culture medium as defined in claims 7 and 10 to 12,
- adding at least one substance or at least one mixture of substances, which may potentially exert an influence on protozoa cells,
- determining the protozoa cell count in said protozoa culture,
- comparing said protozoa cell count before and after adding said at least one substance or at least one mixture of substances.

16. A method for detecting a protozoa species selected from the group consisting of *Histomonas meleagridis, Tetratrichomonas gallinarum* and *Blastocystis sp.* in a sample, comprising the steps of:
- providing a sample,
- contacting said sample with at least one oligonucleotide specific for *Histomonas meleagridis, Tetratrichomonas gallinarum* or *Blastocystis sp.,* or an antibody directed against *Histomonas meleagridis, Tetratrichomonas gallinarum* or *Blastocystis sp.,*
- optionally, subjecting said sample/oligonucleotide mixture to a nucleic acid amplification technique,
- detecting a nucleic acid amplification product, an oligonucleotide/protozoa cell bond or a protozoa cell/antibody bond.

17. The method according to claim 16, **characterized in that** said oligonucleotides 5'-GAAAGCATCTATCAAGTGGAA-3' and 5'-GATCTTTTCAAATTAGCTTTAAA-3' are used for determining *Histomonas meleagridis,* 5'-GCAATTGTTTCTCCAGAAGTG-3' and 5'-GATGGCTCTCTTTGAGCTTG-3' for determining *Tetratrichomonas gallinarum,* and 5'-TAACCGTAGTAATTCTAGGGC-3' and 5'-AACGTTAATATACGCTATTGG-3' for determining *Blastocystis sp..*

18. A set for detecting *Histomonas meleagridis, Tetratrichomonas gallinarum* and *Blastocystis sp.* or antibody directed against any of these cells in a sample comprising:
a) clonal protozoa cells of a single protozoa species selected from the group consisting of *Histomonas meleagridis, Tetratrichomonas gallinarum* and *Blastocystis sp.* or an antibody directed against protozoa cells of a single protozoa species selected from the group consisting of *Histomonas meleagridis, Tetratrichomonas gallinarum* and *Blastocystis sp.,* and
b) means for detecting said protozoa cell/antibody bond.

19. A feedingstuff or feedingstuff additive comprising a vaccine according to any of claims 3 to 6.

## Revendications

1. Culture clonale de protozoaires comprenant des cellules de protozoaires d'une unique espèce de protozoaires, où l'espèce de protozoaires est choisie dans le groupe constitué par *Histomonas meleagridis, Tetratrichomonas gallinarurn* et *Blastocystis sp.,* **caractérisée en ce que** la culture renferme des bactéries symbiotiques ou leur lysat.

2. Culture de protozoaires selon la revendication 1, **caractérisée en ce que** les bactéries sont des entérobactéries, notamment des bactéries coliformes, des staphylocoques, des streptocoques ou leurs mélanges.

3. Formulation vaccinale comprenant des cellules clonales de protozoaires inactivées ou vivantes d'une unique espèce de protozoaires choisie dans le groupe constitué par *Histomonas meleagridis, Tetratrichomonas gallinarum* et *Blastocystis sp.* ou un anticorps dirigé contre les cellules clonales de protozoaires d'une unique espèce de protozoaires choisie dans le groupe constitué par *Histomonas meleagridis, Tetratrichomonas gallinarum* et *Blastocystis sp..*

4. Formulation vaccinale selon la revendication 3, **caractérisée en ce que** le vaccin comprend un adjuvant, qui est choisi de préférence dans le groupe constitué par l'adjuvant complet de Freund, l'adjuvant incomplet de Freund, l'hydroxyde d'aluminium, *Bordetella pertussis,* la saponine, le dipeptide de muramyle, le copolymère d'éthylène - acétate de vinyle, une huile, de préférence une huile végétale ou une huile minérale, en particulier l'huile d'arachide ou l'huile de silicone, et leurs combinaisons.

5. Formulation vaccinale selon la revendication 3 ou 4, **caractérisée en ce que** les cellules clonales de protozoaires sont inactivées par passage, par irradiation, en particulier par irradiation aux UV et radioactive, par des substances chimiques ou par des virus ou par la chaleur, le formol, la bêta-propiolactone ou leurs combinaisons.

6. Formulation vaccinale selon l'une des revendications 3 à 5, **caractérisée en ce que** le vaccin est adapté à l'administration par voie intraveineuse, sous-cutanée, intramusculaire, orale ou intracloacale.

7. Procédé de préparation d'une culture clonale de protozoaires comprenant des cellules de protozoaires d'une unique espèce de protozoaires choisie dans le groupe constitué par *Histomonas meleagridis, Tetratrichomonas gallinarum* et *Blastocystis sp.,* comprenant les étapes consistant à :
a) fournir un échantillon de départ comprenant des cellules de protozoaires d'au moins une espèce de protozoaires choisie dans le groupe constitué par *Histomonas meleagridis, Tetratrichomonas gallinarum* et *Blastocystis sp.,*
b) cultiver les cellules de l'échantillon de départ dans un milieu de culture comprenant une source de glucides et des bactéries d'au moins une espèce bactérienne, qui vivent en symbiose avec les cellules de protozoaires *in situ,* ou un lysat de celles-ci,
c) isoler les cellules de protozoaires au moyen de la micromanipulation,
d) cultiver les cellules de protozoaires isolées dans un milieu de culture tel que défini au b) et
e) le cas échéant déterminer la pureté de la culture clonale de protozoaires.

8. Procédé selon la revendication 7, **caractérisé en ce que** l'échantillon de départ est un échantillon de selles, de tissus, de culture ou de l'environnement.

9. Procédé selon la revendication 7 ou 8, **caractérisé en ce que** l'échantillon de départ vient d'une volaille choisie dans le groupe constitué par la dinde, le poulet, le faisan, le paon, la caille et la pintade.

10. Procédé selon l'une des revendications 7 à 9, **caractérisé en ce que** la source de glucides est l'amidon, en particulier l'amidon de riz, de maïs ou de pommes de terre.

11. Procédé selon l'une des revendications 7 à 10, **caractérisé en ce que** les bactéries sont des entérobactéries, notamment des bactéries coliformes, des staphylocoques, des streptocoques ou leurs mélanges.

12. Procédé selon l'une des revendications 7 à 11, **caractérisé en ce que** le milieu de culture comprend en outre des sels, en particulier des sels d'Earle, des acides aminés, notamment la L-glutamine, et des antibiotiques/antimycosiques, en particulier de la pénicilline, de la streptomycine et de l'amphotéricine.

13. Procédé selon l'une des revendications 7 à 12, **caractérisé en ce que** la pureté de la culture est déterminée au moyen d'une technique d'amplification d'acides nucléiques ou d'hybridation ou au moyen d'une technique immunologique avec des anticorps dirigés contre les cellules clonales de protozoaires d'une unique espèce de protozoaires choisie dans le groupe constitué par *Histomonas meleagridis, Tetratrichomonas gallinarum* et *Blastocystis sp..*

14. Procédé selon la revendication 13, **caractérisé en ce que** les oligonucléotides 5'-GAAAGCATCTATCAAGTGGAA-3' et 5'-GATCTTTTCAAATTAGCTTTAAA-3' sont utilisés pour la détermination de *Histomonas meleagridis,* 5'-GCAATTGTTTCTCCAGAAGTG-3' et 5'-GATGGCTCTCTTTGAGCTTG-3' pour la détermination de *Tetratrichomonas gallinarum,* 5'-TAACCGTAGTAATTCTAGGGC-3' et 5'-AACGTTAATATACGCTATTGG-3' pour la détermination de *Blastocystis sp..*

15. Procédé de détermination de l'impact de substances ou de mélanges de substances sur les cellules de protozoaires d'une espèce de protozoaires choisie dans le groupe constitué par *Histomonas meleagridis, Tetratrichomonas gallinarum* et *Blastocystis sp.,* comprenant les étapes consistant à :
- fournir une culture clonale de protozoaires selon la revendication 1 ou 2,
- le cas échéant enclencher le comptage cellulaire de la culture de protozoaires,
- mettre en contact et cultiver la culture clonale de protozoaires avec un milieu de culture tel que défini dans les revendications 7 et 10 à 12,
- ajouter au moins une substance ou au moins un mélange de substances, qui peut potentiellement avoir un impact sur les cellules de protozoaires,
- déterminer le nombre de cellules de protozoaires dans la culture de protozoaires,
- comparer le nombre de cellules de protozoaires avant et après addition de la au moins une substance ou du au moins un mélange de substances.

16. Procédé de détection d'une espèce de protozoaires choisie dans le groupe constitué par *Histomonas meleagridis, Tetratrichomonas gallinarum* et *Blastocystis sp.* dans un échantillon, comprenant les étapes consistant à :
- fournir un échantillon,
- mettre en contact l'échantillon avec au moins un oligonucléotide spécifique *d'Histomonas meleagridis,* de *Tetratrichomonas gallinarum* ou de *Blastocystis sp.* ou un anticorps dirigé contre *Histomonas meleagridis, Tetratrichomonas gallinarum* ou *Blastocystis sp.,*
- le cas échéant soumettre l'échantillon/le mélange d'oligonucléotides à une technique d'amplification d'acides nucléiques,
- détecter un produit d'amplification d'acides nucléiques, une liaison oligonucléotide/ cellules de protozoaires ou une liaison cellules de protozoaires/ anticorps.

17. Procédé selon la revendication 16, **caractérisé en ce que** les oligonucléotides 5'-GAAAGCATCTATCAAGTGGAA-3' et 5'-GATCTTTTCAAATTAGCTTTAAA-3' sont utilisés pour la détermination de *Histomonas meleagridis,* 5'-GCAATTGTTTCTCCAGAAGTG-3' et 5'-GATGGCTCTCTTTGAGCTTG-3' pour la détermination de *Tetratrichomonas gallinarum,* 5'-TAACCGTAGTAATTCTAGGGC-3' et 5'-AACGTTAATATACGCTATTGG-3' pour la détermination de *Blastocystis sp..*

18. Kit de détection de *Histomonas meleagridis, Tetratrichomonas gallinarum* et *Blastocystis sp.* ou des anticorps dirigés contre l'une de ces cellules dans un échantillon, comprenant :
a) des cellules clonales de protozoaires d'une unique espèce de protozoaires choisie dans le groupe constitué par *Histomonas meleagridis, Tetratrichomonas gallinarum* et *Blastocystis sp.* ou un anticorps dirigé contre les cellules de protozoaires d'une unique espèce de protozoaires choisie dans le groupe constitué par *Histomonas meleagridis, Tetratrichomonas gallinarum* et *Blastocystis sp.* et
b) des moyens de détection de la liaison cellules de protozoaires/ anticorps.

19. Aliments pour animaux ou additif pour l'alimentation animale comprenant un vaccin selon l'une des revendications 3 à 6.
